# EUROPEAN PATENT SPECIFICATION

(11) **EP 2 542 599 B1**
(45) Date of publication and mention of the grant of the patent: **18.12.2013**
(21) Application number: 11705897.4
(22) Date of filing: 01.03.2011
(51) Int. Cl.: C08F 220/06, C08F 226/10, A61K 8/81

(54) **ANIONIC ASSOCIATIVE RHEOLOGY MODIFIERS**
ANIONISCHE ASSOZIATIVE RHEOLOGIEMODIFIKATOREN
MODIFICATEURS ANIONIQUES ASSOCIATIFS DE RHÉOLOGIE

(30) Priority: 02.03.2010 EP 10155173
(43) Date of publication of application: 09.01.2013
(73) Proprietor: BASF SE, 67056 Ludwigshafen (DE)
(72) Inventor: NGUYEN KIM, Son, 69502 Hemsbach (DE); FAST, Ina, 67227 Frankenthal (DE); WERNER, Rolf, 67069 Ludwigshafen (DE)
(74) Representative: Gittinger, Andreas
(86) International application number: PCT/EP2011/053005
(87) International publication number: WO 2011/107463

(56) References cited:
- WO-A1-2007/010035

## Description

The present invention relates to new associative rheology modifiers, their manufacture and application.

Specific requirements are often placed on cosmetic, pharmaceutical and technical compositions with regard to their rheological properties. They can often only be converted to the desired application form using additives, so-called thickeners. Examples of customary low molecular weight thickeners are, for example, alkali metal and aluminum salts of fatty acids, fatty alcohols or waxes. However, depending on the field of use of the preparation to be thickened, the use of known thickeners is often associated with disadvantages. For example, the thickening effect of such thickeners may not be satisfactory or their incorporation into the preparation may be hindered or completely impossible, for example due to incompatibility. The provision of products with a complex profile of properties using the lowest possible fraction or the fewest possible different active substances often presents difficulties. For example, there is a need for polymers for cosmetic compositions which have good conditioning or film-forming properties and at the same time may serve as thickeners for such compositions, In addition, esthetic requirements are increasingly being placed on cosmetic and pharmaceutical products by consumers. For example, with such products, a preference for clear formulations in the form of gels is currently observed. There is therefore a need for cosmetically and pharmaceutically compatible polymers which are suitable for providing a certain property profile with regard to sensory, setting capabilities and rheology.

In general, polymeric thickeners allow the viscosity to be adjusted depending on the polymers' molecular weight. One disadvantage which often arises when using polymers as thickeners for preparing more highly viscous or gel-like preparations is that as the molecular weight of the polymer increases, its incorporation into the cosmetic composition generally becomes more difficult, and that ultimately often only swelling of the polymer is observed instead of the desired solution.

Polymeric rheology modifiers may be classified as either naturally, or synthetically derived products. Examples of the former include starch, cellulose, alginate, and proteins. These naturally occurring polymers incorporate building blocks of polysaccharide units, or amino acids, to provide efficient, water soluble rheology modifiers. Grafting of selected moieties onto the backbone of the more widely utilized natural products, such as starch and cellulose, provides for numerous modified versions of the products, developed to address specific application requirements. Three general classes of acrylic-based synthetic polymers comprise a group of products that have been utilized as rheology modifiers in various applications for many years. The first class is based on homopolymers of (meth)acrylic acid and copolymers of (meth)acrylic acid, (meth)acrylate esters, and maleic acid, among many others. This group is typically referred to as the alkali swellable (or soluble) emulsions (ASE). Modification of the structure of ASE polymers by addition of hydrophobic moieties defines the second class of synthetic rheology modifiers known as the hydrophobically modified, alkali swellable emulsions (HASE). This group of polymers, more commonly referred to as associative thickeners, provides the potential for greater control of the compound rheology, over a broader range of shear rates than the traditional ASE class of synthetic polymers. The third class of synthetic rheology modifiers is the hydrophobically modified, ethoxylated urethane resins (HEUR). This group of polymers typically consists of polyethylene glycol units of varying length, connected by urethane linkages, and terminated with hydrophobic end groups. Unlike the ASE and HASE classes, HEUR rheology modifiers are nonionic substances, and are not dependent on pH for activation of the thickening mechanism.

WO 93/22358 (BASF) describes copolymers obtainable by free-radical polymerization of A) 50-99.99% by weight of an olefinically unsaturated C₃-C₅ monocarboxylic acid, of an olefinically unsaturated C₄-C₈ dicarboxylic acid or the anhydride thereof or a mixture of such carboxylic acids or anhydrides with B) 0.1-29.95% by weight of an olefinically unsaturated quaternary ammonium compound of the formula I or II 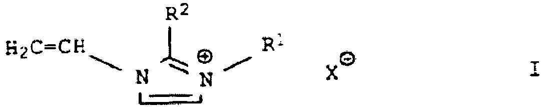 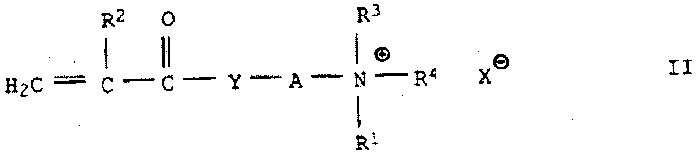 where R¹ is C₆-C₂₀ -alkyl, C₆-C₂₀ -alkenyl, C₅ -C₈-cycloalkyl, phenyl, phenyl(C₁-C₁₂-alkyl) or (C₁-C₁₂-alkyl)phenyl, R² is hydrogen, methyl or phenyl, R³ and R⁴ are each H or C₁-C₄ -alkyl, X is halogen, C₁-C₄-alkoxysulfonyloxy or C₁-C₄-alkanesulfonate, it also being possible for the latter to occur as R³ or R⁴ with the formation of a betaine structure, Y is O or NH, and A is C₁-C₆ -alkylene, or a mixture of such ammonium compounds, C) 0 to 49.99% by weight of an acrylate or methacrylate of the formula III 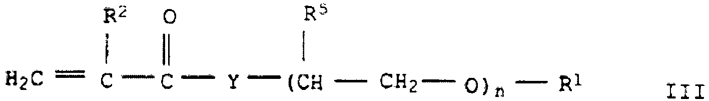 where R¹, R² and Y have the aforementioned meanings, R⁵ is hydrogen, methyl or ethyl, and n is a number from 0 to 25, D) 0-29.85% by weight of other copolymerizable monomers and E) 0.5-2% by weight of one or more compounds with at least two olefinically unsaturated groups in the molecule as crosslinker.

WO 00/39176 (BF Goodrich) describes a hydrophilic ampholytic polymer formed by copolymerization of 0.05 to 20 mole percent of an anionic monomer having at least one carboxyfunctional group, 10 to 45 mole percent of a cationic monomer having at least one aminofunctional group, 35 to 95 mole percent of a nonionic hydrophilic monomer, 0 to 10 mole percent of a hydrophobic monomer and 0 to 1.5 mole percent of a crosslinking monomer, and wherein the monomers are selected so as to provide the copolymer with a glass transition temperature of above about 50°C and the cationic monomer and the anionic monomer are present in a ratio of from about 2 to about 16.

WO 01/62809 (BASF) describes cosmetic compositions comprising at least one water-soluble or water-dispersible polymer which comprises, in incorporated form, a) 5 to 50% by weight of at least one α,β-ethylenically unsaturated monomer of the formula I 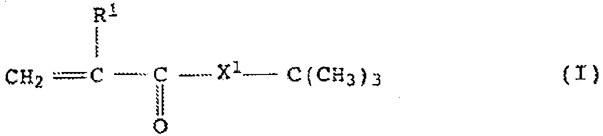 in which R¹ is hydrogen or C₁- to C₈-alkyl, and X¹ is O or NR², where R² is hydrogen, C₁- to C₈-alkyl or C₅-to C₈-cycloalkyl, b) 25 to 90% by weight of at least one N-vinylamide and/or N-vinyllactam, c) 0.5 to 30% by weight of at least one compound having a free-radically polymerizable, α,β-ethylenically unsaturated double bond and at least one cationogenic and/or cationic group per molecule,d) 0 to 30% by weight of at least one α,β-ethylenically unsaturated monomer of the formula II 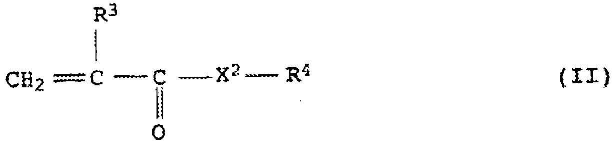 in which R³ is hydrogen or C₁- to C₈-alkyl, X² is O or NR⁵, where R⁵ is hydrogen C₁- to C₈-alkyl or C₅-to C₈-cycloalkyl, and R⁴ is hydrogen or a linear C₁- to C₂₂-alkyl radical and the salts thereof.

WO 03/053381 (BASF) describes cosmetic compositions which comprise at least one water-soluble or water-dispersible copolymer which is obtainable by free-radical copolymerization of at least one N-vinyllactam, at least one anionogenic monomer and optionally further α,β-ethylenically unsaturated compounds copolymerizable therewith, in the presence of a polymer component with repeat units which have ether groups or which are derived from vinyl alcohol.

WO 2004/058837 (BASF) describes cosmetic or pharmaceutical compositions comprising A) at least one ampholytic copolymer obtainable by free-radical copolymerization of a) at least one compound with a free-radically polymerizable, α,β-ethylenically unsaturated double bond and at least one anionogenic and/or anionic group per molecule, b) at least one compound with a free-radically polymerizable, α,β-ethylenically unsaturated double bond and at least one cationogenic and/or cationic group per molecule, c) at least one α,β-ethylenically unsaturated amide-group-containing compound of the formula R¹-CO-NR²R³ in which one of the radicals R¹ to R³ is a group of the formula CH₂=CR⁴, where R⁴= H or C₁-C₄-alkyl, and the other radicals R¹ to R³, independently of one another, are H, alkyl, cycloalkyl, heterocycloalkyl, aryl or hetaryl, where R¹ and R² together with the amide group to which they are bonded may also be a lactam with 5 to 8 ring atoms, where R² and R³ together with the nitrogen atom to which they are bonded may also be a five-to seven-membered heterocycle, with the proviso that the sum of the carbon atoms of the radicals R¹, R² and R³ is at most 8, or a polyelectrolyte complex comprising at least one of said ampholytic copolymers and at least one further polyelectrolyte different therefrom, and B) at least one cosmetically acceptable carrier.

WO 2006/044193 (ISP) and US 2007/0231286 A1 (ISP) describe a rheology modifier/hair styling resin which is a crosslinked, linear poly(vinyl amide/polymerizable carboxylic acid) copolymer and its use in color cosmetic compositions.

WO 2007/010035 A1 (BASF) describes the use of an anionic or cationic ampholytic copolymer which is obtainable by free-radical copolymerization of a1) at least one compound having a free-radically polymerizable, α,β-ethylenically unsaturated double bond and at least one anionogenic and/or anionic group per molecule, a2) at least one compound having a free-radically polymerizable, α,β-ethylenically unsaturated double bond and at least one cationogenic and/or cationic group per molecule, b) at least one free-radically polymerizable crosslinking compound which contains at least two α,β-ethylenically unsaturated double bonds per molecule, c) if desired in the presence of at least one silicone compound containing a polyether group and/or a free-radically polymerizable olefinically unsaturated double bond, as rheology modifiers for compositions in hair cosmetics.

US 4,230,844 (DuPont) describes thickener polymers comprising (i) about 1-99% by weight of at least one unsaturated carboxylic acid of 3 to 6 carbon atoms, and (ii) about 1-99% by weight of at least one ester of the formula 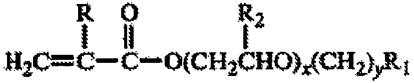 wherein R and R² are each hydrogen or methyl, x is a positive integer of 5 to 80, y is an integer of 0 to 20, and R₁ is alkyl of 1 to 20 carbon atoms or alkyl phenyl where the alkyl group is from 1 to 20 carbon atoms or alkyl phenyl where the alkyl group is from 1 to 20 carbon atoms.

US 4,138,381 (Du Pont) describes thickeners comprising a polymer dissolved in a solvent at a concentration up to 50% by weight of the composition, the solvent being at least one glycol and containing during manufacture up to 50% by weight, based on the weight of glycol, of water and the polymer consists essentially of (a) about 10 to 98% by weight of at least one unsaturated carboxylic acid of 3 to 6 carbon atoms, (b) about 1 to 50% by weight of at least one alkyl acrylate or alkyl methacrylate wherein the alkyl group is from 1 to 30 carbon atoms, and (c) about 1 to 85% by weight of at least one ester of the formula 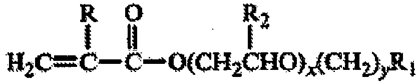 wherein, R and R² are each hydrogen or methyl, x is a positive integer of 5 to 80, y is an integer of 0 to 20, and R₁ is alkyl of 1 to 20 carbon atoms or alkyl phenyl where the alkyl group is from 1 to 20 carbon atoms, the total adding up to 100%.

US 5,015,708 describes non-crosslinked precipitation terpolymer products produced by polymerizing a reaction mixture of a vinyl lactam, e.g. vinyl pyrrolidone or vinyl caprolactam, a polymerizable carboxylic acid, e.g. acrylic acid or methacrylic acid, and a hydrophobic monomer, e.g. lauryl methacrylate, in a predetermined compositional range, in the presence of a polymerization initiator, and in an aliphatic hydrocarbon solvent, particularly a C₃ -C₁₀ saturated hydrocarbon, which is branched or unbranched, cyclic or acylic, and, preferably, is heptane or cyclohexane. The terpolymers are obtained in high yield, as a white powder, which can be filtered and dried easily.

US 6,025,431 describes a polymeric rheology modifier (PRM) which has been prepared by polymerizing from about 5 to about 80 weight percent of an acrylate monomer (a) selected from the group consisting of a C₁-C₆ alkyl ester of acrylic acid and a C₁-C₆ alkyl ester of methacrylic acid, from about 5 to about 80 weight percent of a monomer (b) selected from the group consisting of a vinyl-substituted heterocyclic compound containing at least one of a nitrogen or a sulfur atom, (meth)acrylamide, a mono- or di-(C₁-C₄)alkylamino (C₁-C₄)alkyl (meth)acrylate and a mono or di-(C₁-C₄) alkylamino (C₁-C₄)alkyl (meth)acrylamide, and 0 to about 30 weight percent of an associative monomer (c), all percentages based on the total weight of monomer used to prepare the PRM, and a cosmetically-active agent (CAA).

EP 3235 A1 (BASF) describes a water-soluble copolymer which comprises (a) from 80 to 2% by weight of an ethylenically unsaturated C3- to C5-carboxylic acid, acrylamidodimethylpropanesulfonicacid, vinylsulfonic acid or vinylphosphoric acid, or of an ester of the formula 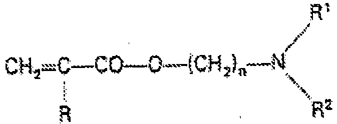 where R is H or-CH₃, n is from 1 to 4 and R¹ and R² are alkyls of 1 to 4 carbon atoms, or of mixtures of the said monomers, and (b) from 20 to 98% by weight of a polymerizable ethylenically unsaturated compound of the formula 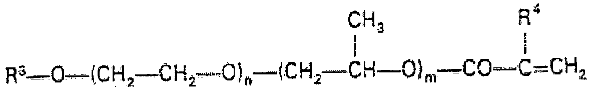 where R³ is alkyl of 1 to 20 carbon atoms, R⁴ is H or -CH₃, R⁵ is H, n is from 2 to 100 and m is from 0 to 50.

EP 11806 A1 (Dow Chemical) describes aqueous liquid emulsion polymers prepared by the copolymerization of (A) 15-60 weight percent of a C₃-C₈ α,β -ethylenically unsaturated carboxylic acid monomer, preferably acrylic or methacrylic acid or a mixture thereof with itaconic or fumaric acid, (B) 15-80 weight percent of a nonionic copolymerizable C₂-C₁₂ α,β-ethylenically unsaturated monomer, preferably a monovinyl ester such as ethyl acrylate or a mixture thereof with styrene, acrylonitrile, vinyl chloride or vinyl acetate, and (C) 1-30 weight percent of certain nonionic vinyl surfactant esters, such as nonylphenoxypoly(ethyleneoxy)₉ ethyl acrylate, to give an emulsion copolymer stable as an aqueous colloidal dispersion at an acid pH lower than about 5.0 but responsive to pH adjustment with base. These emulsion polymers adjusted to a pH of 5.5-10.5 or higher serve as thickeners for aqueous systems including cosmetic products, drilling muds, and particularly aqueous coating compositions such as latex paint.

EP 13836 (Rohm & Haas) describes copolymers containing (1) 20-69.5% by weight of acrylic and/or methacrylic acid; (2) 0.5-25% by weight of monomer of the formula CH₂=C(R)-C(O)-O-(CH₂CH₂O)ₙ-R^{o} wherein R is H or CH₃, n is at least 2 and R^{o} is C₈-C₃₀ alkyl, alkylaryl or polycyclic alkyl; (3) at least 30% by weight of at least one C₁-C₄ alkyl acrylate and/or methacrylate and (4) 0-0.1 % by weight of polyethylenically unsaturated monomer, the total of (1), (2), (3) and (4) being 100%.

EP 1690878 A1 (Rohm & Haas) describes a polymer comprising (a) from 25% to 45% carboxylic acid monomer residues, (b) from 50% to 65% C₂ -C₄ alkyl (meth)acrylate residues, (c) from 2% to 20% of residues of at least one of: (i) an alkyl (meth)acrylate, (ii) a vinyl alkanoate, (iii) an N-vinyl alkylamide, and (iv) an N-alkyl (meth)acrylamide, wherein an alkyl group having from 6-18 carbon atoms is present; and (d) from 0.01% to 2% of residues of at least one crosslinker.

Anionic associative rheologiy modifying polymers are commercially available as e.g. Aculyn^{®}22 (INCI: Acrylates/Steareth-20 Methacrylate Copolymer; a copolymer of the ester of methacrylic acid and Steareth-20 and one or more monomers of acrylic acid, methacrylic acid or one of their simple esters), Aculyn^{®}28 (INCl: Acrylates/Beheneth-25 Methacrylate Copolymer; a copolymer of the ester of methacrylic acid and Beheneth-25 and one or more monomers of acrylic acid, methacrylic acid, or one of their simple esters), Aculyn^{®}88 (INCl: Acrylates/Steareth-20 Methacrylate Crosspolymer; a copolymer of steareth-20 methacrylate and one or more monomers consisting of acrylic acid, methacrylic acid or one of their simple esters, crosslinked with an allyl ether of pentaerythritol or an allyl ether of trimethylolpropane), and Tinovis^{®}GTC (INCl: Acrylates/Beheneth-25 Methacrylate Copolymer).

Although there are plenty of polymeric thickeners for cosmetic use already known and commercially available, there is still a need for thickeners that at the same time can easily be incorporated into cosmetic compositions, enable fast thickening, particularly at pH values in the ranges from 5 to 9, preferrably from 6 to 8, and have good film forming and hair-setting properties. Furthermore, long-term stability of such cosmetic compositions combined with tolerance of comparably high salt contents are desired properties of up-to-date polymeric thickeners. Other desired properties are the ability to form clear gels, stabilizing properties for particles, emulsions, and foams, broad pH range stability, peroxide compatibility, shear tolerance, cold-processable, and broad compatibility with other ingredients.

Surprisingly, it has now been found that these objects are achieved by a polymer comprising as polymerized units
a) 25 to 85 % by weight of acrylic acid,
b) 10 to 60 % by weight of N-vinyl pyrrolidone,
c) 0.5 to 10 % by weight of at least one cationic monomer,
d) 0.5 to 20 % by weight of at least one of compounds d1) or d2)

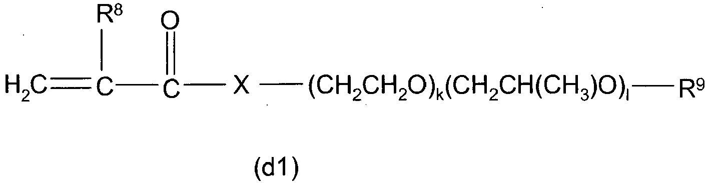

   H₂C=CH-CH₂-O-(CH₂CH₂O)ₖ(CH₂CH(CH₃)O)ₗ-R⁹ (d2)

   wherein
   the order of the alkylene oxide units is arbitrary,
   k and l, independently of one another, are an integer of from 0 to 1000, where the sum of k and l is at least 5,
   R⁸ is hydrogen or C₁-C₄-alkyl, preferably methyl,
   R⁹ is C₈-C₃₀-alkyl, C₈-C₃₀-alkenyl or C₈-C₃₀ alkylaryl, and
   X is O or a group of the formula NR¹⁰, in which R¹⁰ is H, alkyl, alkenyl, cycloalkyl, heterocycloalkyl, aryl or hetaryl,
e) 0.01 to 2 % by weight of at least one crosslinking agent,
f) 0 to 30 % by weight of further monomers different from a) to e), the total of a) to f) adding up to 100 % by weight.

For the purposes of this invention, the term "alkyl" comprises straight-chain and branched alkyl groups. Suitable short-chain alkyl groups are, for example, straight-chain or branched C₁-C₇-alkyl groups, preferably C₁-C₆-alkyl groups and particularly preferably C₁-C₄-alkyl groups. These include, in particular, methyl, ethyl, propyl, isopropyl, n-butyl, 2-butyl, sec-butyl, tert-butyl, n-pentyl, 2-pentyl, 2-methylbutyl, 3-methylbutyl, 1,2-dimethylpropyl, 1,1-dimethylpropyl, 2,2-dimethylpropyl, 1-ethylpropyl, n-hexyl, 2-hexyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-2-methylpropyl, n-heptyl, 2-heptyl, 3-heptyl, 2-ethylpentyl, 1-propylbutyl, octyl.

Suitable longer-chain C₈-C₃₀-alkyl groups and C₈-C₃₀-alkenyl groups are straight-chain and branched alkyl groups and alkenyl groups. Preference is given here to predominantly linear alkyl radicals as also occur in natural or synthetic fatty acids and fatty alcohols and in oxo alcohols, which may, if appropriate, additionally be mono-, di- or polyunsaturated. These include, for example, n-hexyl(ene), n-heptyl(ene), n-octyl(ene), n-nonyl(ene), n-decyl(ene), n-undecyl(ene), n-dodecyl(ene), n-tridecyl(ene), n-tetradecyl(ene), n-pentadecyl(ene), n-hexadecyl(ene), n-heptadecyl(ene), n-octadecyl(ene), n-nonadecyl(ene), arachinyl(ene), behenyl(ene), lignocerinyl(ene), melissinyl(ene).

Cycloalkyl is preferably C₅-C₈-cycloalkyl, such as cyclopentyl, cyclohexyl, cycloheptyl or cyclooctyl.

Aryl comprises unsubstituted and substituted aryl groups and is preferably phenyl, tolyl, xylyl, mesityl, naphthyl, fluorenyl, anthracenyl, phenanthrenyl, naphthacenyl and in particular phenyl, tolyl, xylyl or mesityl.

In the text below, compounds which are derived from acrylic acid and methacrylic acid may sometimes be referred to in short by adding the syllable "(meth)" to the compound derived from acrylic acid.

### Monomer a)

In one embodiment of the invention the polymer comprises as polymerized units of from 25 to 80 % by weight of acrylic acid.
In another embodiment of the invention the polymer comprises as polymerized units of from 40 to 70 % by weight of acrylic acid.
In another embodiment of the invention the polymer comprises as polymerized units of from 40 to 60 % by weight of acrylic acid.

### Monomer b)

In one embodiment of the invention the polymer comprises as polymerized units of from 10 to 60 % by weight of N-vinyl pyrrolidone.
In another embodiment of the invention the polymer comprises as polymerized units of from 20 to 50 % by weight of N-vinyl pyrrolidone.
In another embodiment of the invention the polymer comprises as polymerized units of from 30 to 50 % by weight of N-vinyl pyrrolidone.

### Monomer c)

In one embodiment of the invention the polymer comprises as polymerized units of from 1 to 10 % by weight of at least one cationic monomer.
According to this invention the term "cationic monomer" means monomers carrying a cationic charge or monomers carrying groups, prefarably amine groups, that can be cationically charged either by protonation or by quaternization with acids or alkylating agents, respectively. In other words, the term "cationic monomer" refers to both cationically charged monomers and monomers that can be cationically charged.
In one embodiment of the invention monomer c) comprises at least one compound which is chosen from esters of α,β-ethylenically unsaturated mono- and dicarboxylic acids with amino alcohols which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds and mixtures thereof.

Preferred monomers c) are N-tert-butylaminoethyl (meth)acrylate, N,N-dimethylaminomethyl (meth)acrylate, N,N-dimethylaminoethyl (meth)acrylate, N,N-diethylaminoethyl (meth)acrylate, N,N-dimethylaminopropyl (meth)acrylate, N,N-diethylaminopropyl (meth)acrylate and N,N-dimethylaminocyclohexyl (meth)acrylate. Particular preference is given to N-tert-butylaminoethyl (meth)acrylate and N,N-dimethylaminoethyl (meth)acrylate.

Other preferred monomers c) are, for example, N-[tert-butylaminoethyl(meth)acrylamide, N-[2-dimethylamino)ethyl]acrylamide, N-[2-(dimethylamino)ethyl]methacrylamide, N-[3-(dimethylamino)propyl]acrylamide, N-[3-(dimethylamino)propyl]methacrylamide, N-[4-(dimethylamino)butyl]acrylamide, N-[4-(dimethylamino)butyl]methacrylamide, N-[2-(diethylamino)ethyl]acrylamide, N-[4-(dimethylamino)cyclohexyl]acrylamide and N-[4-(dimethylamino)cyclohexyl]methacrylamide. Particular preference is given to N-[3-(dimethylamino)propyl]acrylamide and N-[3-(dimethylamino)propyl]meth-acrylamide (DMAPMAM).

A specific embodiment relates to polymers which comprise as monomer c) N-[3-dimethylamino)propyl](meth)acrylamide. In another embodiment, monomer c) consists of N-[3-(dimethylamino)propyl](meth)acrylamide.

Other preferred monomers c) are N,N-diallylamines and N,N-diallyl-N-alkylamines and acid addition salts thereof and quaternization products. Alkyl here is preferably C₁-C₂₄-alkyl. Preference is given to N,N-diallyl-N-methylamine and N,N-diallyl-N,N-dimethylammonium compounds, such as, for example, the chlorides and bromides. Particular preference is given to N,N-diallyl-N-methylamine.

Other preferred monomers c) are vinyl- and allyl-substituted nitrogen heterocycles different from vinylimidazoles, such as 2- and 4-vinylpyridine, 2- and 4-allylpyridine, and the salts thereof.

In a preferred embodiment of the invention monomer c) comprises as vinyl-substituted heteroaromatic compound c) at least one N-vinylimidazole compound.
In a specific embodiment of the invention monomer c) is chosen from N-vinylimidazole compounds and mixtures which comprise at least one N-vinylimidazole compound.
In one embodiment of the invention at least one cationic monomer c) is chosen from vinylimidazole compounds of the general formula (II) 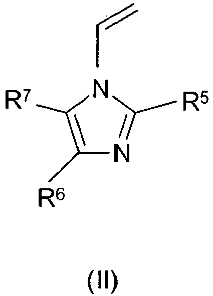 wherein R⁵ to R⁷, independently of one another, are hydrogen, C₁-C₄-alkyl or phenyl.

Examples of compounds c) of the general formula (II) are given in Table 1 below:

**Table 1**

| **R⁵** | **R⁶** | **R⁷** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = methyl Ph = phenyl | | |

In a preferred embodiment of the invention, monomer c) is selected from 1-vinylimidazole (N-vinylimidazole) and mixtures comprising N-vinylimidazole. A particularly preferred embodiment of the invention relates to polymers in which monomer c) consists of N-vinylimidazole.

In one embodiment of the invention the polymer comprises as polymerized units of from 0.5 to 10 % by weight of at least one cationic monomer c).
In another embodiment of the invention the polymer comprises as polymerized units of from 1 to 4 % by weight of at least one cationic monomer c).
In another embodiment of the invention the polymer comprises as polymerized units of from 2 to 6 % by weight of at least one cationic monomer c).

In one embodiment of the invention the molar ratio between monomer a) and monomer c) is at least 4:1. In another embodiment of the invention the molar ratio between monomer a) and monomer c) is at least 10:1. In still another embodiment of the invention the molar ratio between monomer a) and monomer c) is at least 14:1. In still another embodiment of the invention the molar ratio between monomer a) and monomer c) is at least 22:1.
In one embodiment of the invention the molar ratio between monomer a) and monomer c) is at most 110:1. In another embodiment of the invention the molar ratio between monomer a) and monomer c) is at most 80:1. In another embodiment of the invention the molar ratio between monomer a) and monomer c) is at most 50:1.
In a preferred embodiment of the invention the molar ratio between monomer a) and monomer c) is in the range of from 4:1 to 110:1, more preferred in the range of from 10:1 to 80:1, still more preferred in the range of from 14:1 to 50:1.
Preferably, the cationogenic and/or cationic groups of the monomer c) are nitrogen-containing groups, such as primary, secondary and tertiary amino groups, and quaternary ammonium groups. The nitrogen-containing groups are preferably tertiary amino groups or quaternary ammonium groups. Charged cationic groups can be produced from the amine nitrogens either by protonation with acids or by quaternization with alkylating agents. The acids include, for example, carboxylic acids, such as lactic acid, or mineral acids, such as phosphoric acid, sulfuric acid and hydrochloric acid. Alkylating agents are e.g. C₁-C₄-alkyl halides or sulfates, such as ethyl chloride, ethyl bromide, methyl chloride, methyl bromide, dimethyl sulfate and diethyl sulfate. A protonation or quaternization can generally take place either before or preferably after the polymerization.
In one embodiment of this invention, monomers c) are co-polymerized in their non-quaternized form and remain non-quaternized after the polymerization.
In another embodiment of this invention, monomers c) are co-polymerized in their non-quaternized form and at partly quaternized after the polymerization.
In still another embodiment of this invention, monomers c) are co-polymerized in their non-quaternized form and completely quaternized after the polymerization.
However, due to various experimental reasons, there can always be minor portions of monomers c) that are already cationic (i.e. protonated or quaternized) before or during the polymerization although no purposeful protonation/quaternization has been performed.
For reasons of simplicity, throughout this invention the names of the non-ionic, i.e. uncharged monomers c) like e.g. "N-vinylimidazole", "N-[3-(dimethylamino)propyl](meth)acrylamide" or "N,N-diallylamine" stand for both the uncharged and the cationically charged monomers c).

### Monomer d)

The polymers according to this invention comprise as polymerized units of from 0.5 to 10 % by weight of at least one of compounds d1) or d2). In one preferred embodiment of this invention the polymers comprise as polymerized units of from 1 to 6 % by weight of at least one of compounds d1) or d2). In still another preferred embodiment of this invention the polymers comprise as polymerized units of from 2 to 4 % by weight of at least one of compounds d1) or d2).

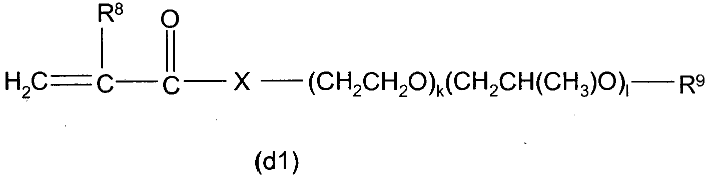

H₂C=CH-CH₂-O-(CH₂CH₂O)ₖ(CH₂CH(CH₃)O)ₗ-R⁹ (d2)

wherein
the order of the alkylene oxide units is arbitrary,
k and l, independently of one another, are an integer from 0 to 1000, where the sum of k and l is at least 5,
R⁸ is hydrogen or C₁-C₄-alkyl, preferably methyl,
R⁹ is C₈-C₃₀-alkyl, C₈-C₃₀-alkenyl or C₈-C₃₀ alkylaryl, and
X is O or a group of the formula NR¹⁰, in which R¹⁰ is H, alkyl, alkenyl, cycloalkyl, heterocycloalkyl, aryl or hetaryl.

In one preferred embodiment of the invention, R⁸ is hydrogen, methyl, ethyl, n-propyl, isopropyl, n-butyl, sec-butyl, tert-butyl, n-pentyl or n-hexyl, particularly preferred hydrogen, methyl or ethyl. Most preferably R⁸ is methyl.

In one preferred embodiment of the invention, k is an integer in the range of from 1 to 500, in particular of from 3 to 250 and l is an integer in the range of from 0 to 100.

In one preferred embodiment of the invention, R⁹ of monomers d1) and d2) is selected from n-octyl, 1,1,3,3-tetramethylbutyl, ethylhexyl, n-nonyl, n-decyl, n-undecyl, tridecyl, myristyl, pentadecyl, palmityl, heptadecyl, octadecyl, nonadecyl, arrachinyl, behenyl, lignocerenyl, cerotinyl, melissinyl, palmitoleinyl, oleyl, linolyl, linolenyl, stearyl, lauryl. In particularly preferred embodiments of this invention R⁹ of monomers d1) and d2) is selected from palmityl, heptadecyl, octadecyl, nonadecyl, arrachinyl, behenyl and their binary or tertiary mixtures. In another preferred embodiment of this invention R⁹ of monomers d1) and d2) is selected from the binary and tertiary mixtures of the even-numbered alkyl residues like e.g. C₁₆, C₁₈, C₂₀, and C₂₂.

Preferably, X in formula d1) is O or NH.

Suitable polyether (meth)acrylates d1) are, for example, the condensation products of (meth)acrylic acid with polyetherols. Suitable polyetherols can be prepared easily by reacting ethylene oxide, 1,2-propylene oxide and/or epichlorohydrin with a starter alcohol R⁹-OH. The alkylene oxides can be used individually, alternately one after the other or as a mixture. The polyether (meth)acrylates d1) can be used on their own or in mixtures for the preparation of the polymers according to the invention.

In one embodiment of this invention the polymers according to this invention comprise as polymerized units at least one compound d1) chosen from polyether (meth)acrylates terminated with C₈-C₂₂-alkyl groups.

Preferred monomers d1) according to this invention are esters of methacrylic acid with ethoxylated C₁₆-C₁₈ alkyl alcohols, wherein the degree of ethoxylation (k in formula d1)) is 10 to 40, preferably 20 to 30.
In one particularly preferred embodiment of the invention, the commercially available C₁₈-PEG 1100 MA (Plex^{®}6877-O, CAS number 70879-51-5 (APG 1100 MA), manufacturer: Degussa) is selected as at least one monomer d1).

In another embodiment of the invention, monomer d1) can be prepared as described in U.S. Pat. No. 3,708,445 using alcohols and acids described in column 3, lines 36-75. This particular monomer d1) is of the formula: 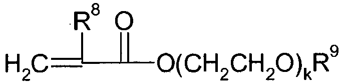 wherein R⁸ is hydrogen or methyl,
k is a positive integer of 5 to 80, preferably 10 to 50, and
R⁹ is alkyl of 8 to 15 carbon atoms or alkyl phenyl wherein the alkyl group is from 8 to 20 carbon atoms, preferably 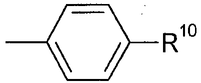 wherein R¹⁰ is alkyl of 8 to 20 carbon atoms.

Suitable allyl alcohol alkoxylates d2) are, for example, the etherification products of allyl chloride with corresponding polyetherols. Suitable polyetherols can be prepared easily by reacting ethylene oxide, 1,2-propylene oxide and/or epichlorohydrin with water or a starter alcohol R⁹-OH. The alkylene oxides can be used individually, alternately one after the other or as a mixture. The allyl alcohol alkoxylates d2) can be used on their own or in mixtures for the preparation of the polymers according to this invention.

### Monomer e)

The polymers according to this invention comprise in copolymerized form of from 0.01 to 2 % by weight of at least one crosslinking agent (crosslinker) e), i.e. a compound with two or more ethylenically unsaturated, nonconjugated double bonds.

Suitable crosslinkers e) are e.g. (meth)acrylic esters, allyl ethers or vinyl ethers of at least dihydric alcohols. The OH groups of the parent alcohols here may be completely or partially etherified or esterified; however, the crosslinkers comprise at least two ethylenically unsaturated groups.

Examples of the parent alcohols are dihydric alcohols, such as 1,2-ethanediol, 1,2-propanediol, 1,3-propanediol, 1,2-butanediol, 1,3-butanediol, 2,3-butanediol, 1,4-butanediol, but-2-ene-1,4-diol, 1,2-pentanediol, 1,5-pentanediol, 1,2-hexanediol, 1,6-hexanediol, 1,10-decanediol, 1,2-dodecanediol, 1,12-dodecanediol, neopentyl glycol, 3-methylpentane-1,5-diol, 2,5-dimethyl-1,3-hexanediol, 2,2,4-trimethyl-1,3-pentanediol, 1,2-cyclohexanediol, 1,4-cyclohexanediol, 1,4-bis(hydroxymethyl)cyclohexane, hydroxypivalic neopentyl glycol monoester, 2,2-bis(4-hydroxyphenyl)propane, 2,2-bis[4-(2-hydroxypropyl)phenyl]propane, diethylene glycol, triethylene glycol, tetraethylene glycol, dipropylene glycol, tripropylene glycol, tetrapropylene glycol, 3-thiopentane-1,5-diol, and polyethylene glycols, polypropylene glycols and polytetrahydrofurans with molecular weights of in each case 200 to 10000. Apart from the homopolymers of ethylene oxide and propylene oxide also block copolymers of ethylene oxide or propylene oxide or copolymers which comprise incorporated ethylene oxide and propylene oxide groups can be used. Examples of parent alcohols with more than two OH groups are trimethylolpropane, glycerol, pentaerythritol, 1,2,5-pentanetriol, 1,2,6-hexanetriol, triethoxycyanuric acid, sorbitan, sugars, such as sucrose, glucose, mannose. The polyhydric alcohols can also be used following reaction with ethylene oxide or propylene oxide as the corresponding ethoxylates or propoxylates. The polyhydric alcohols can also firstly be converted into the corresponding glycidyl ethers by reaction with epichlorohydrin. Preference is given to ethylene glycol di(meth)acrylate and polyethylene glycol di(meth)acrylates.

Further suitable crosslinkers e) are the vinyl esters or the esters of monohydric, unsaturated alcohols with ethylenically unsaturated C₃-C₆-carboxylic acids, for example acrylic acid, methacrylic acid, itaconic acid, maleic acid or fumaric acid. Examples of such alcohols are allyl alcohol, 1-buten-3-ol, 5-hexen-1-ol, 1-octen-3-ol, 9-decen-1-ol, dicyclopentenyl alcohol, 10-undecen-1-ol, cinnamyl alcohol, citronellol, crotyl alcohol or cis-9-octadecen-1-ol. However, it is also possible to esterify the mono-hydric, unsaturated alcohols with polybasic carboxylic acids, for example malonic acid, tartaric acid, trimellitic acid, phthalic acid, terephthalic acid, citric acid or succinic acid.

Further suitable crosslinkers e) are esters of unsaturated carboxylic acids with the above-described polyhydric alcohols, for example of oleic acid, crotonic acid, cinnamic acid or 10-undecenoic acid.

Suitable crosslinkers e) are also straight-chain or branched, linear or cyclic, aliphatic or aromatic hydrocarbons which have at least two double bonds which, in the case of aliphatic hydrocarbons, must not be conjugated, e.g. divinylbenzene, divinyltoluene, 1,7-octadiene, 1,9-decadiene, 4-vinyl-1-cyclohexene, trivinylcyclohexane or polybutadienes with molecular weights of from 200 to 20000.

Also suitable as crosslinkers e) are the acrylamides, methacrylamides and N-allylamines of at least difunctional amines. Such amines are, for example, 1,2-diaminomethane, 1,2-diaminoethane, 1,3-diaminopropane, 1,4-diaminobutane, 1,6-diaminohexane, 1,12-dodecanediamine, piperazine, diethylenetriamine or iso-phoronediamine. Likewise suitable are the amides of allylamine and unsaturated carboxylic acids, such as acrylic acid, methacrylic acid, itaconic acid, maleic acid, or at least dibasic carboxylic acids as have been described above.

In addition, triallylamine and triallylmonoalkylammonium salts, e.g. triallylmethylammonium chloride or methyl sulfate, are suitable as crosslinker e).

Also suitable are N-vinyl compounds of urea derivatives, at least difunctional amides, cyanurates or urethanes, for example of urea, ethyleneurea, propyleneurea or tartardiamide, e.g. N,N'-divinylethyleneurea or N,N'-divinylpropyleneurea.

Further suitable crosslinkers e) are divinyldioxane, tetraallylsilane or tetravinylsilane.

It is of course also possible to use mixtures of the abovementioned compounds e).

In one preferred embodiment of this invention, crosslinker e) is at least one of ethylene glycol di(meth)acrylate, polyethylene glycol di(meth)acrylates, pentaerythritol triallyl ether, methylenebisacrylamide, N,N'-divinylethyleneurea, triallylamine and triallylmonoalkylammonium salts.

In one preferred embodiment of this invention, crosslinker e) is pentaerythritol triallyl ether.

### Monomer f)

The polymers according to this invention comprise as polymerized units of from 0 to 30 % by weight of further monomers f) different from a) to e). In one embodiment of the invention, the polymers comprise as polymerized units of from 1 to 20 % by weight of such further monomers f). In still another embodiment of the invention, the polymers comprise as polymerized units of from 2 to 10 % by weight of such further monomers f).

In one embodiment of the invention, at least one monomer f) is chosen from the group consisting of methyl (meth)acrylate, ethyl (meth)acrylate, n-propyl (meth)acrylate, isopropyl (meth)acrylate, methyl ethacrylate, ethyl ethacrylate, n-propyl ethacrylate, isopropyl ethacrylate, n-butyl ethacrylate, tert-butyl ethacrylate, isobutyl ethacrylate, n-butyl (meth)acrylate, tert-butyl (meth)acrylate, isobutyl (meth)acrylate, sec-butyl (meth)acrylate, 2-pentyl (meth)acrylate, 3-pentyl (meth)acrylate, isopentyl acrylate, neopentyl acrylate, n-octyl (meth)acrylate, 1,1,3,3-tetramethylbutyl (meth)acrylate, ethylhexyl (meth)acrylate, n-nonyl (meth)acrylate, n-decyl (meth)acrylate, n-undecyl (meth)acrylate, tridecyl (meth)acrylate, myristyl (meth)acrylate, pentadecyl (meth)acrylate, palmityl (meth)acrylate, heptadecyl (meth)acrylate, nonadecyl (meth)acrylate, arrachinyl (meth)acrylate, behenyl (meth)acrylate, lignocerenyl (meth)acrylate, cerotinyl (meth)acrylate, melissinyl (meth)acrylate, palmitoleinyl (meth)acrylate, oleyl (meth)acrylate, linolyl (meth)acrylate, linolenyl (meth)acyrlate, stearyl (meth)acrylate, lauryl (meth)acrylate, phenoxyethyl acrylate, 4-t-butylcyclohexyl acrylate, cyclohexyl (meth)acrylate, ureido (meth)acrylate, tetrahydrofurfuryl (meth)acrylate and mixtures thereof.
One preferred embodiment of the invention is a polymer as described before, wherein at least one monomer f) is chosen from esters of α,β-ethylenically unsaturated mono-and dicarboxylic acids with C₁-C₈-alkanols.
Preferably, at least one monomer f) is chosen from C₁-C₆ alkyl (meth)acrylates, still more preferred C₁-C₄ alkyl (meth)acrylates, e.g. from the group consisting of methyl (meth)acrylate, ethyl (meth)acrylate, n-propyl (meth)acrylate, isopropyl (meth)acrylate, methyl ethacrylate, ethyl ethacrylate, n-propyl ethacrylate, isopropyl ethacrylate, n-butyl ethacrylate, tert-butyl ethacrylate, isobutyl ethacrylate, n-butyl (meth)acrylate, tert-butyl (meth)acrylate, isobutyl (meth)acrylate, sec-butyl (meth)acrylate.
In one preferred embodiment of the invention, at least one monomer f) is chosen from the group consisting of methyl methacrylate, ethyl methacrylate, n-propyl methacrylate, isopropyl methacrylate, n-butyl methacrylate, tert-butyl methacrylate, isobutyl methacrylate, sec-butyl methacrylate and mixtures thereof.
In one particularly preferred embodiment of the invention, at least one monomer f) is or comprises methyl methacrylate (MMA).

In another embodiment of the invention, at least one monomer f) is chosen from compounds which are different from N-vinyl pyrrolidone and of the general formula VI 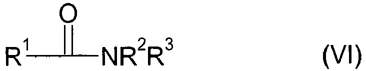 where R¹ is a group of the formula CH₂=CR⁴- where R⁴ = H or C₁-C₄-alkyl and R² and R³ are, independently of one another, H, alkyl, cycloalkyl, heterocycloalkyl, aryl or hetaryl or R² and R³ together with the nitrogen atom to which they are bonded are a 5-to 8-membered nitrogen heterocycle or
R² is a group of the formula CH₂=CR⁴- and R¹ and R³ are, independently of one another, H, alkyl, cycloalkyl, heterocycloalkyl, aryl or hetaryl or R¹ and R³ together with the amide group to which they are bonded are a lactam having 5 to 8 ring atoms.

In one embodiment of the invention, preferred monomers f) are N-vinyllactams. Suitable monomers f) are unsubstituted N-vinyllactams and N-vinyllactam derivatives, which can, for example, have one or more C₁-C₆-alkyl substituents, such as methyl, ethyl, n-propyl, isopropyl, n-butyl, sec-butyl, tert-butyl etc. These include, for example, N-vinylpiperidone, N-vinylcaprolactam, N-vinyl-5-methyl-2-pyrrolidone, N-vinyl-5-ethyl-2-pyrrolidone, N-vinyl-6-methyl-2-piperidone, N-vinyl-6-ethyl-2-piperidone, N-vinyl-7-methyl-2-caprolactam, N-vinyl-7-ethyl-2-caprolactam etc. and mixtures thereof.

In one embodiment of the invention, preferred monomers f) are N-vinylcaprolactam, N-vinylformamide, acrylamide, methacrylamide, tert-butylacrylamide, N,N-dimethylacrylamide or mixtures thereof.

In still another embodiment of the invention suitable monomers f) are the amides of (meth)acrylic acid different from c). Such amides are, for example, N-methyl(meth)acrylamide, N-ethyl(meth)acrylamide, N-n-propyl(meth)acrylamide, N-i-propyl(meth)acrylamide, N-(n-butyl)(meth)acrylamide, N-(sec-butyl)(meth)acrylamide, N-(tert-butyl)methacrylamide, N-(n-pentyl)(meth)acrylamide, N-(n-hexyl)(meth)acrylamide, N-(n-heptyl)(meth)acrylamide, N-(n-octyl)(meth)acrylamide, N-(tert-octyl)(meth)acrylamide N-(1,1,3,3-tetramethylbutyl)(meth)acrylamide, N-ethylhexyl(meth)acrylamide, N-(n-nonyl)(meth)acrylamide, N-(n-decyl)(meth)acrylamide, N-(n-undecyl)(meth)acrylamide, N-tridecyl(meth)acrylamide, N-myristyl(meth)acrylamide, N-pentadecyl(meth)acrylamide, N-palmityl(meth)acrylamide, N-heptadecyl(meth)acrylamide, N-nonadecyl(meth)acryl-amide, N-arrachinyl(meth)acrylamide, N-behenyl(meth)acrylamide, N-lignocerenyl-(meth)acrylamide, N-cerotinyl(meth)acrylamide, N-melissinyl(meth)acrylamide, N-palmitoleinyl(meth)acrylamide, N-oleyl(meth)acrylamide, N-linolyl(meth)acrylamide, N-linolenyl(meth)acrylamide, N-stearyl(meth)acrylamide, N-lauryl(meth)acrylamide.

In still another embodiment of the invention suitable monomers f) are 2-hydroxyethylacrylamide, 2-hydroxyethylmethacrylamide, 2-hydroxyethylethacrylamide, 2-hydroxypropylacrylamide, 2-hydroxypropylmethacrylamide, 3-hydroxypropylacrylamide, 3-hydroxypropylmethacrylamide, 3-hydroxybutylacrylamide, 3-hydroxybutylmethacrylamide, 4-hydroxybutylacrylamide, 4-hydroxybutylmethacrylamide, 6-hydroxyhexylacrylamide, 6-hydroxyhexylmethacrylamide, 3-hydroxy-2-ethylhexylacrylamide and 3-hydroxy-2-ethylhexylmethacrylamide.

In still another embodiment of the invention, at least one monomer f) is chosen from vinyl acetate, vinyl propionate, vinyl butyrate, ethylene, propylene, isobutylene, butadiene, styrene, α-methylstyrene, acrylonitrile, methacrylonitrile, vinyl chloride, vinylidene chloride, vinyl fluoride, vinylidene fluoride and mixtures thereof.

In still another embodiment of the invention, at least one monomer f) different from acrylic acid is chosen from monomers with a free-radically polymerizable, α,β-ethylenically unsaturated double bond and at least one anionogenic and/or anionic group per molecule. Such monomers f) include monoethylenically unsaturated mono- and dicarboxylic acids having 3 to 25, preferably 3 to 6, carbon atoms, which can also be used in the form of their salts or anhydrides. Examples thereof are methacrylic acid, ethacrylic acid, α-chloroacrylic acid, crotonic acid, maleic acid, maleic anhydride, itaconic acid, citraconic acid, mesaconic acid, glutaconic acid, aconitic acid and fumaric acid. Such monomers f) also include the half-esters of monoethylenically unsaturated dicarboxylic acids having 4 to 10, preferably 4 to 6, carbon atoms, e.g. of maleic acid, such as monomethyl maleate. Such monomers f) also include monoethylenically unsaturated sulfonic acids and phosphonic acids, for example vinylsulfonic acid, allylsulfonic acid, sulfoethyl acrylate, sulfoethyl methacrylate, sulfopropyl acrylate, sulfopropyl methacrylate, 2-hydroxy-3-acryloxypropylsulfonic acid, 2-hydroxy-3-methacryloxypropylsulfonic acid, styrenesulfonic acid, 2-acrylamido-2-methylpropanesulfonic acid, vinylphosphonic acid and allylphosphonic acid. Such monomers f) also include the salts of the abovementioned acids, in particular the sodium, potassium and ammonium salts, and the salts with amines. Such monomers f) can be used as they are or as mixtures with one another. The weight fractions given all refer to the acid form.
In one embodiment of the invention the component f) is chosen from methacrylic acid, ethacrylic acid, α-chloroacrylic acid, crotonic acid, maleic acid, maleic anhydride, fumaric acid, itaconic acid, citraconic acid, mesaconic acid, glutaconic acid, aconitic acid, 2-acrylamido-2-methylpropanesulfonic acid, vinylphosphonic acid and mixtures thereof.

In one preferred embodiment of the invention, component f) is chosen from methacrylic acid, ethacrylic acid, α-chloroacrylic acid, crotonic acid, maleic acid, maleic anhydride, fumaric acid, itaconic acid, citraconic acid, mesaconic acid, glutaconic acid, aconitic acid and mixtures thereof.

In one particulary preferred embodiment of the invention, component f) is or comprises methacrylic acid.

In one embodiment of the invention, for the manufacture of the polymers, monomers d) and f) are used as mixtures. Such mixtures of monomers d) and f) are for example mixtures comprising C₁₈-(EO)₂₅ methacrylate and methyl methacrylate (like e.g. Piex^{®}6877-O) or mixtures comprising C₁₈-(EO)₂₅ methacrylate and methacrylic acid (like e.g. Lutencryl^{®}250).

The following table shows exemplary compositional ranges of monomers a) to f) for polymers according to this invention. The numbers are in % by weight with the proviso that a) to f) add up to a total of 100 % by weight; a) is acrylic acid, b) is N-vinyl pyrrolidone, c) is preferably N-vinyl imidazole, d) is preferably C₁₆-C₁₈ PEG-25 methacrylate, i.e. monomer d) according to formula d1) wherein R⁸ is Methyl, X is Oxygen, k is about 25, l is zero, R⁹ is a mixture of C₁₆ alkyl and C₁₈ alkyl; e) is preferably pentaerythritol triallyl ether (PETAE); and f) is preferably methyl methacrylate and/or methacrylic acid.

| Polymer No | a) | b) | c) | d) | e) | f) |
|---|---|---|---|---|---|---|
| I | 54-60 | 28-33 | 1-4 | 2-3 | 0.2-0.7 | 6-9 |
| II | 48-52 | 34-38 | 2-4 | 2-3 | 0.3-0.8 | 6-9 |
| III | 38-42 | 46-50 | 2-4 | 1-3 | 0.3-0.8 | 4-8 |
| IV | 30-34 | 56-60 | 2-4 | 1-2 | 0.4-0.8 | 3-5 |
| V | 38-42 | 38-42 | 8-10 | 1-3 | 0.4-0.8 | 6-9 |
| VI | 38-42 | 36-40 | 4-8 | 7-9 | 0.5-1.2 | 6-10 |
| VII | 65-75 | 15-25 | 0.5-3 | 2-6 | 0.2-1.0 | 1-6 |
| VIII | 75-85 | 10-18 | 0.5-2 | 1-6 | 0.2-1.0 | 1-6 |
| IX | 45-60 | 30-50 | 1-3 | 1-5 | 0.2-1.0 | 2-5 |

One embodiment of the invention are polymers comprising as polymerized units
a) 30 to 70 % by weight of acrylic acid,
b) 25 to 60 % by weight of N-vinyl pyrrolidone,
c) 2 to 10 % by weight of monomer c), preferably N-vinyl imidazole,
d) 1 to 9 % by weight of monomer d), preferably C₁₆-C₁₈ PEG-25 methacrylate,
e) 0.1 to 1.5 % by weight of at least one crosslinking agent e), preferably PETAE,
f) 1 to 15 % by weight of further monomers different from a) to e),
the total of a) to f) adding up to 100 % by weight.

Another embodiment of the invention are polymers comprising as polymerized units
a) 30 to 50 % by weight, preferably 35 to 45 % by weight of acrylic acid,
b) 30 to 50 % by weight, preferably 35 to 45 % by weight of N-vinyl pyrrolidone,
c) 2 to 8 % by weight of monomer c), preferably N-vinyl imidazole,
d) 3 to 9 % by weight of monomer d), preferably C₁₆-C₁₈ PEG-25 methacrylate,
e) 0.5 to 1.5 % by weight of at least one crosslinking agent e), preferably PETAE,
f) 2 to 10 % by weight, preferably 4 to 8 % by weight of further monomers different from a) to e), preferably methacrylic acid and/or methylmethacrylate, the total of a) to f) adding up to 100 % by weight.

Another embodiment of the invention are polymers comprising as polymerized units
a) 35 to 45 % by weight of acrylic acid,
b) 40 to 55 % by weight of N-vinyl pyrrolidone,
c) 2 to 8 % by weight of monomer c), preferably N-vinyl imidazole,
d) 2 to 6 % by weight of monomer d), preferably C₁₆-C₁₈ PEG-25 methacrylate,
e) 0.5 to 1.5 % by weight of at least one crosslinking agent e), preferably PETAE,
f) 4 to 15 % by weight, preferably 4 to 10 % by weight of methylmethacrylate,
the total of a) to f) adding up to 100 % by weight.

Another embodiment of the invention are polymers comprising as polymerized units
a) 65 to 75 % by weight of acrylic acid,
b) 10 to 25 % by weight of N-vinyl pyrrolidone,
c) 0.5 to 2 % by weight of monomer c), preferably N-vinyl imidazole,
d) 2 to 6 % by weight of monomer d), preferably C₁₆-C₁₈ PEG-25 methacrylate,
e) 0.5 to 1.5 % by weight of at least one crosslinking agent e), preferably PETAE,
f) 2 to 8 % by weight, preferably 3 to 6 % by weight of methacrylic acid and 0.2 to 1.0 % by weight of methylmethacrylate,
the total of a) to f) adding up to 100 % by weight.

Another embodiment of the invention are polymers comprising as polymerized units
a) 70 to 80 % by weight of acrylic acid,
b) 10 to 18 % by weight of N-vinyl pyrrolidone,
c) 0.5 to 3 % by weight of monomer c), preferably N-vinyl imidazole,
d) 2 to 6 % by weight of monomer d), preferably C₁₆-C₁₈ PEG-25 methacrylate,
e) 0.5 to 1.5 % by weight of at least one crosslinking agent e), preferably PETAE,
f) 2 to 6 % by weight, preferably 3 to 6 % by weight of methacrylic acid and 0.2 to 1.0 % by weight of methylmethacrylate,
the total of a) to f) adding up to 100 % by weight.

### Precipitation Polymerization

The polymers according to the invention are manufactured by the method of precipitation polymerization.
The invention thus further provides a method for manufacturing the polymers according to the invention wherein the polymerization is a precipitation polymerization.

In a specific embodiment of the precipitation polymerization use is made of at least two free-radical initiators whose decomposition temperatures and/or half-lives thereof at a certain polymerization temperature are different from one another. As a result, copolymers with particularly low residual monomer contents can be achieved. This is the case, particularly if the initiator that decomposes at the higher temperature is added before the polymer has finished precipitating, preferably before the polymer has started precipitating.

During precipitation polymerization the monomers are soluble in the reaction medium which comprises the monomers and the solvent but not the resulting polymer. The resulting polymer becomes insoluble under such polymerization conditions and precipitates. Thereby it is possible to obtain copolymers with higher molecular weights compared to other polymerization processes, e.g. through solution polymerization. Such copolymers having relatively high molecular weights are particularly advantageous as rheology modifiers, in particular as thickeners.

The precipitation polymerization preferably takes place in a solvent, in which each of the monomers used is soluble (at 20°C and 1 bar) in an amount of at least 10% by weight to give a solution visibly clear to the human eye.
The precipitation polymerization takes place, for example, in esters such as ethyl acetate or butyl acetate and/or hydrocarbons such as cyclohexane or n-heptane as solvents. In one embodiment of the invention mixtures of ethyl acetate and cyclohexane are used as solvent.
The resulting polymer particles precipitate from the reaction solution and may be isolated by known methods such as filtration at reduced pressure.

The polymerization temperatures are preferably in a range from about 30 to 120°C, particularly preferably from 40 to 100°C.

Suitable Initiators for the free-radical polymerization are peroxo and/or azo compounds customary for this purpose, for example alkali metal or ammonium peroxydisulfates, diacetyl peroxide, dibenzoyl peroxide, succinyl peroxide, di-tert-butyl peroxide, tert-butyl perbenzoate, tert-butyl perpivalate, tert-butyl peroxy-2-ethylhexanoate, tert-butyl permaleate, cumene hydroperoxide, diisopropyl peroxydicarbamate, bis(o-toluoyl) peroxide, didecanoyl peroxide, dioctanoyl peroxide, dilauroyl peroxide, tert-butyl perisobutyrate, tert-butyl peracetate, di-tert-amyl peroxide, tert-butyl hydroperoxide, 2,2'-Azobis(2.4-dimethyl valeronitrile), Azobis(2-amidinopropane) dihydrochloride, 2-2'-Azobis(2-methylbutyronitrile) (Wako^{®}V65), tert.butyl peroctoate (CAS No 13467-82-8), 2,5-Dimethyl-2,5-bis(t-butylperoxy)hexane (Trigonox^{®}101).

Also suitable are initiator mixtures or redox initiator systems, such as, for example, ascorbic acid/iron(II) sulfate/sodium peroxodisulfate, tert-butyl hydroperoxide/sodium disulfite, tert-butyl hydroperoxide/sodium hydroxymethanesulfinate, H₂O₂/Cu(I).

To produce polymers with minimized residual monomer contents, the first polymerization (main polymerization) may be followed by an afterpolymerization step. For such afterpolymerization the same or a different initiator system as for the main polymerization may be used. Preferably the temperature of the afterpolymerization step is equal to, preferably higher than the main polymerization temperature. The reaction temperature during the main polymerization is preferably at most 100°C and during the afterpolymerization preferably at most and 130°C.

In a specific embodiment at least two free radical initiators which permit an essentially independent initiation in at least two phases are used for the preparation of the polymers according to the invention. Thereby polymers with particularly low residual monomer contents can be achieved.
US 2008/0199416 A1, [0494] to [0508], which is herewith incorporated by reference, gives a detailed description of such a kind of process.

After polymerization the precipitated polymer is isolated from the reaction mixture. Therefore any method known to the skilled person can be used. Such methods are filtration, centrifugation, evaporation of the solvent or combinations of these methods. The polymers can be further purified by conventional washing steps with the same solvents that have been used for the polymerization itself.

Resulting dry polymer powders can advantageously be converted to an aqueous solution or dispersion by dissolution or redispersion, respectively, in water. Pulverulent copolymers have the advantage of better storability and easier transportability and usually exhibit a lower propensity for microbial attack.

The acid groups of the polymers can be partially or completely neutralized with a base. Bases which can be used for the neutralization of the polymers are alkali metal bases such as sodium hydroxide solution, potassium hydroxide solution, sodium carbonate, sodium hydrogencarbonate, potassium carbonate, potassium hydrogencarbonate, ammonium bicarbonate, ammonium carbonate, and alkaline earth metal bases, such as calcium hydroxide, calcium oxide, magnesium hydroxide, magnesium carbonate and also amines. Suitable amines are, for example, C₁-C₆-alkylamines, preferably n-propylamine and n-butylamine, dialkylamines, preferably diethylpropylamine and dipropylmethylamine, trialkylamines, preferably triethylamine and triisopropylamine. Preference is given to amino alcohols, e.g. trialkanolamines, such as triethanolamine, alkyldialkanolamines, such as methyl- or ethyldiethanolamine and dialkylalkanolamines, such as dimethylethanolamine, and also 2-amino-2-methyl-1-propanol. Particularly for use in hair treatment compositions, 2-amino-2-methyl-1-propanol, 2-amino-2-ethylpropane-1,3-diol, diethylaminopropylamine and triisopropanolamine have proven particularly useful for the neutralization of the polymers comprising acid groups. The neutralization of the acid groups can also be carried out with the aid of mixtures of two or more bases, e.g. mixtures of sodium hydroxide solution and triisopropanolamine. Depending on the intended used, the neutralization can take place partially or completely.

In one preferred embodiment of the invention the precipitation polymerization is carried out in the presence of at least one surfactant. Preferably, this surfactant has an HLB value of less than or equal to 10.
Using the HLB-value (according to W.C.Griffin, J. Soc. Cosmetic Chem. 1 (1949) 311), emulsifiers can be classified according to the ratio of hydrophilic groups to lipophilic groups (HLB = hydrophilic-lipophilic balance).
Suitable surfactants with an HLB value of less than or equal to 10 are described, for example, in Karl-Heinz Schrader, Grundlagen und Rezepturen der Kosmetika [Fundamentals and Formulations of Cosmetics], 2nd edition, Verlag Hüthig, Heidelberg, pp. 395 - 397, to which reference is made here in its entirety.
The determination of the HLB value of emulsifiers is known to the person skilled in the art and is described, for example, on p. 394 of the abovementioned literature reference. Further suitable surfactants with an HLB value of less than or equal to 10 are listed, for example, in US 4375533, column 7, II. 26 - 60, to which reference is made here in its entirety. The use of surfactants in the precipitation polymerization of crosslinked polyacrylic acid is already described in US 4420596 and US 4375533.

The invention thus also provides a method as described above where the precipitation polymerization is carried out in the presence of at least one surfactant which is chosen from linear block copolymers with a hydrophobic structural unit with a length of more than 5 nm (calculated by the law of cosinés), which are defined by the following formula:

C_{w}-(B-A-B_{y})ₓ-D_{z}

in which
A is a hydrophilic structural unit which has a solubility in water at 25°C of 1 % by weight or more, has a molar mass M_{w} of from 200 to 50 000, and is selected such that it is bonded covalently to B;
B is a hydrophobic structural unit which has a molar mass M_{w} of from 300 to 60 000, has a solubility in water at 25°C of less than 1 % by weight and can be covalently bonded to A;
C and D are end groups which may be A or B and the same group or different groups; w is 0 or 1;
x is an integer greater than or equal to 1,
y is 0 or 1, and
z is 0 or 1.
The invention further provides a method as described above where the precipitation polymerization is carried out in the presence of at least one surfactant chosen from random comb copolymers which are defined by the following formula:

R₁-(Z)ₘ-(Q)ₙ-R₂

in which
R₁ and R₂ are end groups and may be identical or different from one another and are different from Z and Q,
Z is a hydrophobic structural unit which has a solubility in water of 25°C of less than 1 % by weight,
Q is a hydrophilic structural unit which has a solubility in water of 25°C of more than 1 % by weight,
   and
m and n are integers greater than or equal to 1 and are selected such that the molar mass M_{w} is from 100 to 250 000.

In one preferred embodiment of the invention the surfactants are selected from 12-hydroxystearic acid block copolymers, further preferably 12-hydroxystearic acid block copolymers with polyethylene oxide. The 12-hydroxystearic acid block copolymers are particularly preferably ABA block copolymers.
1) Hypermer^{®} B239: block copolymer of a polyhydroxy fatty acid (PFA) and polyethylene oxide (PEO) with Mw of about 3500;
2) Hypermer^{®} B246: block copolymer of a polyhydroxy fatty acid (PFA) and polyethylene oxide (PEO) with Mw of about 7500.
3) Hypermer^{®} B261: block copolymer of a polyhydroxy fatty acid (PFA) and polyethylene oxide (PEO) with Mw of about 9600.
4) Hypermer^{®} 2234: nonionic polymeric surface-active compound;
5) Hypermer^{®} LP6: polymeric fatty acid ester with M_{w} of about 4300.
6) Hypermer^{®} IL2296: nonionic polymeric surface-active compound;
7) Hypermer^{®} A-1 09 block copolymer of a fatty acid or of a long-chain alkylene radical with ethylene oxide.
8) Hypermer^{®} A-409 block copolymer of a fatty acid or of a long-chain alkylene radical with ethylene oxide.
9) Pecosil^{®} PS-100 dimethicone copolyol phosphate polymer with 5-12 mol of ethylene oxide per mole of the hydrophilic unit.
10) Pecosil^{®} WDS-1 00 dimethicone copolyol phosphate polymer with 5-12 mol of propylene oxide per mole of the hydrophilic unit.
Useful surfactants are disclosed in EP 584771 B1, page 23, lines 2 to 37, which is hereby incorporated by reference.
In another embodiment of the invention the surfactant is selected from the group consisting of
- copolymers of polydimethylsiloxanes and organic glycols,
- substances with the INCI name dimethicone PEG-7 phosphate,
- polyesters comprising polyethylene glycol,
- polyoxyethylene-glycerol-fatty-acid esters,
- polyamide waxes,
- natural waxes and
- mixtures thereof.

Other surfactants are copolymers of polydimethylsiloxanes and organic glycols like e.g. substances with the INCI name PEG/PPG-25/25 dimethicone (e.g. Belsil^{®}DMC 6031) and dimethicone copolyol acetate (e.g. Belsil^{®}DMC 6032).
Another suitable commercially available dimethicone PEG-7 phosphate (INCI) is e.g. Pecosil^{®}PS-100.
Other suitable commercially available dimethicones are aminoalkyl subsituted dimethicones like e.g. Methoxy PEG/PPG-7/3 Aminopropyl Dimethicone (Abil^{®}Soft AF 100 by Evonik). Aminoalkyl subsituted surfactants enable higher solid contents during the precipitation polymerization compared to the use of non aminated surfactants.
Commercially available polyesters comprising polyethylene glycol that may be used as surfactants in the precipitation polymerization are block copolymers of the Hypermer^{®} brand, in particular the grades B239, B246, B261, 2234, LP6, A-109, A-409 (described in EP 584771 B1, page10, lines 25-42).

Other surfactants in the presence of which the monomers are polymerized to yield the polymers of this invention are mixtures of different ethoxilated long chain fatty alcohols, like e.g. Leophen^{®}RW ECO.

Commercially available polyoxyethylene-glycerol-fatty-acid esters also suitable as surfactants are e.g. polyglyceryl-2 dipolyhydroxystearates (INCI) such as Dehymuls^{®}PGPH (Cognis).
Other suitable surfactants are compounds with the INCI names PEG-7 Hydrogenated Castor Oil, such as Arlacel^{®}989, Cremophor^{®}W07 (BASF) or Dehymuls^{®}HRE 7 (Cognis), PEG-2 Hydrogenated Castor Oil such as Arlacel^{®}582, sorbitan monooleate/propylglyceryl 4/3-ricinoleate, such as Arlacel^{®}1689 (Croda), sorbitan stearate and sucrose cocoate such as, for example, Arlartone^{®}2121 (Croda), sorbeth-20 beeswax such as, for example, Atlas^{®}G-1726 (Croda).
Another suitable surfactant is a polyamide wax like e.g. Kahl Wax 6635 (Kahl&Co).
Other suitable commercially available natural waxes are e.g. mixtures of fatty acid esters, fatty acid and fatty alcohol, such as, for example, beeswax, berry wax, rice wax (Kahl&Co). Suitable beeswaxes are in particular those with the CAS numbers 8006-40-4 (white) or 8012-89-3. Suitable beeswaxes bear the INCI (EU) names Cera Alba, synthetic beeswax, PEG-7 dimethicone beeswax. Particularly suitable beeswaxes are those with the INCI EU name Cera Alba.
Suitable berry waxes are e.g. those with the INCI name Rhus Verniciflua Peel Wax (Berry Wax 6290 (Kahl & Co) or Botaniwax^{®}OT(Botanigenics, Inc)).
Suitable rice waxes are in particular those with CAS number 8016-60-2 or the INCI name Oryza Sativa (rice) bran wax. Such rice waxes are available commercially as Cerewax^{®} (Chemyunion Quimica LTDA), ESP^{®}Rice Bran Wax (Earth Supplied Products, LLC), Florabeads^{®}RBW(Floratech Americas), Naturebead^{®}R20 (Micro Powders, Inc. Personal Care Division), Oryza Soft^{®}"COS" (Cosmetochem International Ltd.), ORYZA^{®} Wax (Ichimaru Pharcos Company, Ltd.), Ricebran Wax SP 8000 (Strahl & Pitsch, Inc.), Rice Wax No.1(Tri-K Industries), Rice Wax 2811 (Kahl).

The amount of surfactant present during the precipitation polymerization is in the range from 0.001 to 50% by weight, preferably from 0.01 to 20% by weight and particularly preferably in the range from 0.1 to 10% by weight, based on the total amount of 100% by weight of the components a) to f).

On account of their thickening effect, the polymers obtainable by the method according to the invention can be used as the sole gel former in cosmetic preparations. Moreover, they are also suitable for use in combination with customary gel formers.

The polymers according to the invention can be used, in particular as thickeners, in aqueous preparations in the sectors of household, personal care, building industry, textiles, for paper coating slips, pigment printing pastes, aqueous colors, leather-treatment compositions, cosmetic formulations, pharmaceutical products and agrochemicals.

Another embodiment of this invention is a method for modifying the viscosity of aqueous compositions, wherein said method comprises adding a polymer according to this invention to said aqueous compositions.

Furthermore, the polymers of this invention are easy to handle, allow for use of continuous production processes with use of in-line static mixers, can be processed with membrane pumps and, when diluted, with turbine mixers and high speed propellers, are able to formulate clear products, can be used with electrolytes, support the stabilization of hydrophobic (low solubility) components, are compatible with nonionic, anionic, zwitterionic and some cationic surfactants, are able to stabilize suspensions, are mild, soft, non-greasy, non-sticky, stable in pH 5.5 to 12 formulations, thicken and stabilize hydrogen peroxide, and allow a flexible choice of the preservative system.

Another embodiment of this invention are cosmetic preparations, which comprise the polymers according to the invention.
Non-limiting examples for cosmetic preparations where the polymers of this inventions can be advantageously used are anti-dandruff shampoos, bath foams, curl activators, depilatories, emulsifier free formulations, foaming facial cleansers, hair styling gels, liquid soaps, lotions, moisturizing creams, shampoos, shower gels, skin masks, waterless hand cleaners, and wave sets.

### Examples

The invention is illustrated in more detail by reference to the following nonlimiting examples.

### Abbreviations:

- VP: N-Vinyl pyrrolidone
- MAA: Methacrylic acid
- AA: Acrylic acid
- MA: methacrylate
- MMA: methyl methacrylate
- VI: N-Vinyl imidazole
- EAc: Ethyl acetate
- CH: Cyclohexane
- PETAE: Pentaerythritol triallyl ether
- C₁₆₋₁₈-Alkyl-PEG-MA: Methacrylic acid ester of a C₁₆-C₁₈-fatty alcohol alkoxylated with 25 moles of ethylene oxide
- Plex^{®}6877-O:: C₁₆₋₁₈-Alkyl-PEG₁₁₀₀ - methacrylate in methyl methacrylate [25:75 w:w]
- Lutencryl^{®}250:: C₁₆₋₁₈-Alkyl-PEG₁₁₀₀- methacrylate in methacrylic acid (MAS) [50:50 w:w]
- LUMA:: C₁₆₋₁₈Alkyl-PEG₁₁₀₀- methacrylate

### I.) Preparation of polymers

Preferably, the polymers of this invention are manufactured by precipitation polymerization.

### Example 67:

### Copolymer of AA / VP / VI / C₁₆₋₁₈-Alkyl-PEG-MA / PETAE / MAA (40/37/6/8/1/8 w/w)

| Initial charge: | | |
|---|---|---|
| | 1020 g | ethyl acetate (EAc) |
| | 6 g | Belsil^{®} DMC 6031 |
| | | |
| Feed 1: | 150 g | ethyl acetate |
| | 240 g | acrylic acid |
| | 222 g | N-vinyl pyrrolidone |
| | 36 g | N-vinyl imidazole |
| | 96 g | Lutencryl^{®}250 |
| | 8 g | pentaerythritol triallyl ether |
| | | |
| Feed 2 | 300 g | ethyl acetate |
| | 3.31 g | tert-butyl peroctoate |
| | 0.30 g | Wako^{®} V 50 |
| | | |
| Feed 3: | 900 g | ethyl acetate |

A stirred reaction vessel was filled with initial charge, kept under nitrogen atmosphere and the initial charge was heated to 70°C. Feed 1 was then added continuously during 3 hours and Feed 2 was added continuously during 5 hours. After completion of Feed 1, Feed 3 was added continuously during 1.5 hours. After completion of Feed 2, the reaction mixture was heated to 75°C and kept at 75°C for another 3 hours before it was heated to 100°C and kept at 100°C for another 4 hours.

Polymers of examples 1 to 51 and 62 to 97 were prepared in an analogous manner.

For the preparation of the polymers of example 52 to 61, a 1:1 w/w mixture of ethyl acetate and cyclohexane has been used instead of ethyl acetate.
All monomer amounts given in the following table are in weight-%.

| **Polymer No** | **AA** | **VP** | **VI** | **Monomers d) + f)** | **PETAE** | **Surfactant ¹⁾** |
|---|---|---|---|---|---|---|
| | | | | | | |
| 1 | 40 | 48.4 | 3 | 8 ²⁾ | 0.6 | 0 |
| 2 | 40 | 40 | 9.4 | 10 ²⁾ | 0.6 | 0 |
| 3 | 40 | 48.4 | 3 | 8 ²⁾ | 0.6 | 0 |
| 4 | 45 | 43.3 | 3 | 8 ²⁾ | 0.7 | 0 |
| 5 | 45 | 43.3 | 3 | 8 ²⁾ | 0.7 | 1% |
| 6 | 45 | 43.3 | 3 | 8 ²⁾ | 0.5 | 0 |
| 7 | 40 | 48.2 | 3 | 8 ²⁾ | 0.8 | 1% |
| 8 | 40 | 44 | 3 | 12 ²⁾ | 1.0 | 1% |
| 9 | 40 | 44 | 3 | 12 ²⁾ | 1.0 | 1% |
| 10 | 40 | 38 | 6 | 15 ²⁾ | 1.0 | 1% |
| 11 | 40 | 44.3 | 3 | 12 ²⁾ | 0.7 | 1% |
| 12 | 40 | 44.3 | 3 | 12 ²⁾ | 0.7 | 1% |
| 13 | 40 | 45.2 | 6 | 8 ²⁾ | 0.8 | 1% |
| 14 | 40 | 44.3 | 3 | 12 ²⁾ | 0.7 | 1% |
| 15 | 40 | 45 | 6 | 8 ³⁾ | 1.0 | 1% |
| 16 | 40 | 41 | 6 | 12 ³⁾ | 1.0 | 1% |
| 17 | 40 | 37 | 6 | 16 ³⁾ | 1.0 | 1% |
| 18 | 40 | 45 | 6 | 8 ³⁾ | 1.0 | 1% |
| 19 | 40 | 54 | 3 | 2 (LUMA) | 1.0 | 1% |
| 20 | 40 | 50 | 5 | 4 ³⁾ | 1.0 | 1% |
| 21 | 40 | 45.1 | 6 | 8 ³⁾ | 0.9 | 1% |
| 22 | 40 | 37 | 6 | 16 ³⁾ | 1.0 | 1% |
| 23 | 40 | 45 | 6 | 8 ³⁾ | 1.0 | 1% |
| 24 | 34 | 50 | 3 | 12 ²⁾ | 1.0 | 1% |
| 25 | 34 | 54 | 3 | 8 ²⁾ | 1.0 | 1% |
| 26 | 34 | 54 | 3 | 8 ²⁾ | 1.0 | 1% |
| 27 | 40 | 50 | 3 | 6 ²⁾ | 1.0 | 1% |
| 28 | 45 | 40 | 6 | 8 ³⁾ | 1.0 | 1% |
| 29 | 37 | 40 | 6 | 16 ³⁾ | 1.0 | 1% |
| 30 | 38.5 | 50 | 3 | 8 ³⁾ | 0.5 | 1% |
| 31 | 26.5 | 50 | 3 | 20 ²⁾ | 0.5 | 1% |
| 32 | 40 | 46.5 | 3 | 10 ³⁾ | 0.5 | 1% |
| 33 | 30 | 46.5 | 3 | 20 ²⁾ | 0.5 | 1% |
| 34 | 46.5 | 40 | 3 | 10 ³⁾ | 0.5 | 1% |
| 35 | 46.4 | 40 | 3 | 10 ³⁾ | 0.6 | 1% |

| **Polymer No** | **AA** | **VP** | **VI** | **Plex^{®} 6877-O** | **Lutencryl^{®} 250** | **PETAE** | **Surfactant** |
|---|---|---|---|---|---|---|---|
| 36 | 40 | 48.3 | 3 | 8 | 0 | 0.7 | 1% ¹⁾ |
| 37 | 40 | 48.2 | 3 | 8 | 0 | 0.8 | 1% ¹⁾ |
| 38 | 40 | 48.1 | 3 | 8 | 0 | 0.9 | 1% ¹⁾ |
| 39 | 43 | 45 | 3 | 8 | 0 | 1.0 | 1% ¹⁾ |
| 40 | 40 | 45.2 | 6 | 8 | 0 | 0.8 | 1% ¹⁾ |
| 41 | 40 | 48.2 | 3 | 8 | 0 | 0.8 | 1% ¹⁾ |
| 42 | 40 | 46.2 | 3 | 10 | 0 | 0.8 | 1% ¹⁾ |
| 43 | 40 | 44.2 | 3 | 12 | 0 | 0.8 | 1% ¹⁾ |
| 44 | 50 | 34 | 3 | 12 | 0 | 1 | 1% ¹⁾ |
| 45 | 50 | 34 | 3 | 12 | 0 | 1 | 1% ¹⁾ |
| 46 | 45 | 45 | 3 | 6 | 0 | 1 | 1% ¹⁾ |
| 47 | 40 | 48.3 | 3 | 8 | 0 | 0.7 | 1% ¹⁾ |
| 48 | 40 | 48.2 | 3 | 8 | 0 | 0.8 | 1% ¹⁾ |
| 49 | 40 | 48.1 | 3 | 8 | 0 | 0.9 | 1% ¹⁾ |
| 50 | 40 | 45 | 6 | 0 | 8 | 1 | 1% ¹⁾ |
| 51 | 40 | 44.9 | 6 | 0 | 8 | 1.1 | 1% ¹⁾ |
| 52 | 60 | 30.5 | 1 | 0 | 8 | 0.50 | 0 |
| 53 | 65 | 25.5 | 1 | 0 | 8 | 0.50 | 0 |
| 54 | 70 | 20.5 | 1 | 0 | 8 | 0.50 | 0 |
| 55 | 70 | 16.5 | 1 | 0 | 12 | 0.50 | 0 |
| 56 | 70 | 12.5 | 1 | 0 | 16 | 0.50 | 0 |
| 57 | 73.3 | 17 | 1 | 0 | 8 | 0.7 | 1.5% ⁵⁾ |
| 58 | 56.5 | 30 | 3 | 10 | 0 | 0.5 | 1% ¹⁾ |
| 59 | 56.5 | 30 | 3 | 10 | 0 | 0.5 | 2% ⁴⁾ |
| 60 | 56.5 | 30 | 3 | 10 | 0 | 0.5 | 1% ¹⁾ |
| 61 | 54.5 | 30 | 5 | 10 | 0 | 0.5 | 1% ¹⁾ |
| 62 | 54.5 | 30 | 3 | 12 | 0 | 0.5 | 1% ¹⁾ |
| 63 | 50 | 36.4 | 3 | 10 | 0 | 0.6 | 1% ¹⁾ |
| 64 | 40 | 48.4 | 3 | 8 | 0 | 0.6 | 1% ¹⁾ |
| 65 | 30 | 58.4 | 3 | 6 | 0 | 0.6 | 1% ¹⁾ |
| 66 | 40 | 40 | 9.4 | 10 | 0 | 0.6 | 1% ¹⁾ |
| 67 | 40 | 37 | 6 | 0 | 16 | 1.0 | 1% ¹⁾ |
| 68 | 45 | 45 | 2 | 0 | 8 | 0.5^{*)} | 1% ¹⁾ |
| 69 | 45 | 45 | 2 | 0 | 8 | 0.6^{*)} | 1% ¹⁾ |
| 70 | 45 | 45 | 2 | 0 | 8 | 0.5^{*)} | 1% ⁵⁾ |
| 71 | 47 | 47 | 2 | 0 | 4 | 0.6^{*)} | 1% ¹⁾ |
| 72 | 58 | 36 | 2 | 0 | 4 | 0.5^{*)} | 1% ¹⁾ |
| 73 | 60 | 30 | 2 | 0 | 8 | 0.5^{*)} | 1% ¹⁾ |
| 74 | 60 | 30 | 2 | 0 | 8 | 0.5^{*)} | 1% ¹⁾ |
| 75 | 65 | 22 | 2 | 0 | 11 | 0.5^{*)} | 1% ¹⁾ |
| 76 | 66 | 22 | 2 | 0 | 10 | 0.5^{*)} | 1% ¹⁾ |
| 77 | 70 | 22 | 2 | 0 | 6 | 0.5^{*)} | 1% ¹⁾ |
| 78 | 70 | 22 | 2 | 0 | 6 | 0.4^{*)} | 1% ¹⁾ |
| 79 | 70 | 20 | 2 | 0 | 8 | 0.5^{*)} | 0 |
| 80 | 73 | 17 | 2 | 0 | 8 | 0.5^{*)} | 1% ¹⁾ |
| 81 | 73 | 17 | 2 | 0 | 8 | 0.5^{*)} | 1% ⁵⁾ |
| 82 | 74 | 17 | 1 | 0 | 8 | 0.5^{*)} | 1% ¹⁾ |
| 83 | 74 | 17 | 1 | 0 | 8 | 0.4^{*)} | 1% ¹⁾ |
| 84 | 74 | 17 | 1 | 0 | 8 | 0.3^{*)} | 1% ¹⁾ |
| 85 | 74 | 17 | 1 | 0 | 8 | 0.5^{*)} | 1% ⁵⁾ |
| 86 | 74 | 21 | 1 | 0 | 4 | 0.5^{*)} | 1% ¹⁾ |
| 87 | 78 | 17 | 1 | 0 | 4 | 0.5^{*)} | 1% ¹⁾ |
| 88 | 78 | 10 | 1 | 0 | 11 | 0.5^{*)} | 1% ¹⁾ |
| 89 | 79 | 12 | 1 | 0 | 8 | 0.5^{*)} | 1% ¹⁾ |
| 90 | 79 | 12 | 1 | 0 | 8 | 0.5^{*)} | 1% ⁵⁾ |
| 91 | 80 | 11 | 1 | 0 | 8 | 0.5^{*)} | 1% ¹⁾ |
| 92 | 80 | 11 | 1 | 0 | 8 | 0.4^{*)} | 1% ¹⁾ |
| 93 | 80 | 11 | 1 | 0 | 8 | 0.3^{*)} | 1% ⁵⁾ |
| 94 | 80 | 15 | 1 | 0 | 4 | 0.5^{*)} | 1% ¹⁾ |
| 95 | 83 | 12 | 1 | 0 | 4 | 0.5^{*)} | 1% ¹⁾ |
| 96 | 84 | 12 | 1 | 0 | 3 | 0.5^{*)} | 1% ¹⁾ |
| 97 | 85 | 10 | 1 | 0 | 4 | 0.5^{*)} | 1% ¹⁾ |

| | | | | | | | |
|---|---|---|---|---|---|---|---|
| ¹⁾: Belsil^{®} DMC 6031 was used as surfactant; amount given in weight-% with respect to sum of amount of monomers AA, VP, VI, monomer d), f) and PETAE which add up to 100 weight-%; ²⁾: Plex^{®}6877-O was used as monomers d) + f); ³⁾: Lutencryl^{®}250 was used as monomers d) + f); ⁴⁾: Hypermer^{®}B246 was used as surfactant ; amount given in weight-% with respect to sum of amount of monomers AA, VP, VI, monomer d), f) and PETAE which add up to 100 weight-%; ⁵⁾: Abil^{®}Soft AF 100 was used as surfactant; amount given in weight-% with respect to sum of amount of monomers AA, VP, VI, monomer d), f) and PETAE which add up to 100 weight-%; *) amount of PETAE in weight-% with respect to the total amount of all other monomers being 100 % by weight. | | | | | | | |

### II.) Application of polymers

In the following, the term "Polymer selected from No. 1 to 97" means any of the Polymers No 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97.

Hair gels containing a nonionic hair-setting agent:

| | CTFA | % by wt. |
|---|---|---|
| Phase 1: | | |
| Polymer selected from No. 1 to 97 | | 0.5 |
| Water, dist. | | 49.5 |

Further additive: preservative, e.g. Euxyl^{®} K100, perfume, etc.
with triethanolamine (50% strength solution) adjust to pH between 6.7 and 7.4

| Phase 2: | | |
|---|---|---|
| Luviskol^{®} K90 | Polyvinylpyrrolidone | 3.0 |
| Water, dist. | | ad 100 |

Further additives: perfume, emulsifier, UV-absorber, etc.

### Preparation:

Phase 1 is weighed and homogenized with stirring at a temperature in the range from 20 to 50°C. After about 1 to 3 hours, a milky dispersion forms. Triethanolamine is added with stirring. After about 2 hours, a (virtually) homogeneous, high-viscosity gel is formed. Phase 2 is then stirred slowly into phase 1. The gel is stirred at room temperature for a further hour. This produces a stable, nearly clear to clear gel.

### Hair gels containing poly(vinylpyrrolidone/vinyl acetate)

| | CTFA | % by wt. |
|---|---|---|
| Phase 1: | | |
| Polymer selected from No. 1 to 97 | | 0.5 |
| Water, dist. | | 49.5 |

Further additive: preservative, e.g. Euxyl® K100, perfume etc.
with triethanolamine (50% strength solution) adjust to pH between 6.7 - 7.4.

| Phase 2: | | |
|---|---|---|
| Luviskol^{®} VA64 | Poly(vinylpyrrolidone/vinyl acetate) | 4.5 |
| Water, dist. | | ad 100 |

Further additives: perfume, emulsifier, UV-absorber, etc.

### Preparation:

Phase 1 is weighed and homogenized with stirring at a temperature in the range from 20 to 50°C. After about 3 hours, a milky dispersion forms. Triethanolamine is added with stirring. After about 2 hours, a (virtually) homogeneous, high-viscosity gel is formed. Phase 2 is then stirred slowly into phase 1. The gel is stirred at room temperature for a further hour. This produces a stable, nearly clear to clear gel.

### Hair gels containing a cationic hair polymer

| | CTFA | % by wt. |
|---|---|---|
| Phase 1: | | |
| Polymer selected from No. 1 to 97 | | 1.0 |
| Water, dist. | | 49 |

Further additive: preservative, e.g. Euxyl^{®} K100, perfume, etc.
with triethanolamine (50% strength solution) adjust to pH between 6.7-7.2

| Phase 2: | | |
|---|---|---|
| Luviskol^{®} K90 | Polyvinylpyrrolidone | 1.5 |
| Luviquat^{®} Supreme (BASF AG) | Polyquaternium-68 | 0.5 |
| Water, dist. | | ad 100 |

Further additives: perfume, emulsifier, UV-absorber, etc.

### Preparation:

Phase 1 is weighed and homogenized with stirring at a temperature in the range from 20 to 50°C. After about 3 hours, a milky dispersion forms. Triethanolamine is added with stirring. After about 2 hours, a (virtually) homogeneous, high-viscosity gel is formed. Phase 2 is then stirred slowly into phase 1. The gel is stirred at room temperature for a further hour. This produces a stable gel.

### Shampoo (without the addition of salt)

| | CTFA | % by wt. |
|---|---|---|
| Phase 1: | | |
| Polymer selected from No. 1 to 97 | | 1.7 |
| Water | | 47.5 |

with triethanolamine (50% strength) adjust to pH 6 to 7

| Phase 2: | | |
|---|---|---|
| Texapon® NSO 28% strength | Sodium Laureth Sulfate/ Henkel | 50.0 |
| Comperlan® KD | Coamide DEA / Henkel | 1.0 |

Further additive: perfume, preservative, etc.

### Preparation:

Weigh in and, with stirring, dissolve phases 1 and 2 separately and mix. Slowly stir phase 2 into phase 1.

## Claims

1. Polymer comprising as polymerized units
a) 25 to 85 % by weight of acrylic acid,
b) 10 to 60 % by weight of N-vinyl pyrrolidone,
c) 0.5 to 10 % by weight of at least one cationic monomer,
d) 0.5 to 10 % by weight of at least one of compounds d1) or d2) 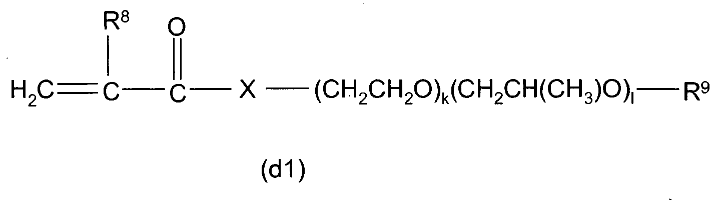
H₂C=CH-CH₂-O-(CH₂CH₂O)ₖ(CH₂CH(CH₃)O)ₗ-R⁹ (d2)
wherein
the order of the alkylene oxide units is arbitrary,
k and l, independently of one another, are an integer from 0 to 1000, where the sum of k and l is at least 5,
R⁸ is hydrogen or C₁-C₄-alkyl,
R⁹is C₈-C₃₀-alkyl, C₈-C₃₀-alkenyl or C₈-C₃₀ alkylaryl, and
X is O or a group of the formula NR¹⁰, in which R¹⁰ is H, alkyl, alkenyl, cycloalkyl, heterocycloalkyl, aryl or hetaryl,
e) 0.01 to 2 % by weight of at least one crosslinking agent,
f) 0 to 30 % by weight of further monomers different from a) to e),
the total of a) to f) adding up to 100 % by weight.

2. Polymer according to claim 1, wherein at least one cationic monomer c) is chosen from vinylimidazole compounds of the general formula (II) 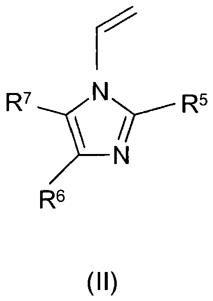 in which R⁵ to R⁷, independently of one another, are hydrogen, C₁-C₄-alkyl or phenyl.

3. Polymer according to claim 2, wherein at least one cationic monomer c) is N-vinyl imidazole.

4. Polymer according to one of claims 1 to 3, wherein at least one monomer f) is chosen from methacrylic acid, esters of α,β-ethylenically unsaturated mono-and dicarboxylic acids with C₁-C₈-alkanols.

5. Polymer according to claim 4, wherein at least one monomer f) is chosen from C₁-C₆-(meth)acrylates.

6. Polymer according to one of claims 1 to 5, wherein at least one compound d1) is chosen from polyether (meth)acrylates terminated with C₈-C₂₂-alkyl groups.

7. Polymer according to one of claims 1 to 6, wherein the molar ratio of compounds a) to c) is at least 4:1.

8. Polymer according to one of claims 1 to 7, comprising as polymerized units
a) 65 to 75 % by weight of acrylic acid,
b) 10 to 25 % by weight of N-vinyl pyrrolidone,
c) 0.5 to 2 % by weight of N-vinyl imidazole,
d) 2 to 6 % by weight of C₁₆-C₁₈ PEG-25 methacrylate,
e) 0.5 to 1.5 % by weight of at least one crosslinking agent,
f) 2 to 8 % by weight of methacrylic acid and 0.2 to 1.0 % by weight of methylmethacrylate,
the total of a) to f) adding up to 100 % by weight.

9. Cosmetic composition comprising a polymer according to one of claims 1 to 8.

10. Method for modifying the viscosity of aqueous compositions, wherein said method comprises adding a polymer according to one of claims 1 to 8 to said aqueous compositions.

## Patentansprüche

1. Polymer, umfassend als polymerisierte Einheiten
a) 25 bis 85 Gew.-% Acrylsäure,
b) 10 bis 60 Gew.-% N-Vinylpyrrolidon,
c) 0,5 bis 10 Gew.-% an wenigstens einem kationischen Monomer,
d) 0,5 bis 10 Gew.-% an wenigstens einer der Verbindungen d1) und d2), 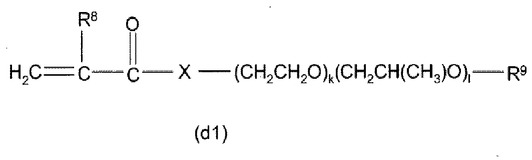
H₂C=CH-CH₂-O-(CH₂CH₂O)ₖ(CH₂CH(CH₃)O)ₗ-R⁹ (d2)
wobei
die Reihenfolge der Alkylenoxideinheiten willkürlich ist,
k und l unabhängig voneinander ganze Zahlen von 0 bis 1000 sind, wobei die Summe von k und l wenigstens 5 beträgt,
R⁸ Wasserstoff oder C₁-C₄-Alkyl ist,
R⁹ C₈-C₃₀-Alkyl, C₈-C₃₀-Alkenyl oder C₈-C₃₀-Alkylaryl ist und
X O oder eine Gruppe der Formel NR¹⁰ ist, wobei R¹⁰ H, Alkyl, Alkenyl, Cycloalkyl, Heterocycloalkyl, Aryl oder Hetaryl ist,
e) 0,01 bis 2 Gew.-% an wenigstens einem Vernetzungsmittel,
f) 0 bis 30 Gew.-% an weiteren Monomeren, die von a) bis e) verschieden sind,
wobei sich die Gesamtmenge von a) bis f) auf 100 Gew.-% aufsummiert.

2. Polymer gemäß Anspruch 1, wobei wenigstens ein kationisches Monomer c) ausgewählt ist aus Vinylimidazolverbindungen der allgemeinen Formel (II), 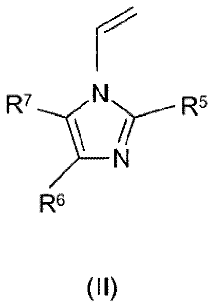 wobei R⁵ bis R⁷ unabhängig voneinander Wasserstoff, C₁-C₄-Alkyl oder Phenyl sind.

3. Polymer gemäß Anspruch 2, wobei wenigstens ein kationisches Monomer c) N-Vinylimidazol ist.

4. Polymer gemäß einem der Ansprüche 1 bis 3, wobei wenigstens ein Monomer f) ausgewählt ist aus Methacrylsäure, Estern von α,β-ethylenisch nichtgesättigten Mono- und Dicarbonsäuren mit C₁-C₈-Alkanolen.

5. Polymer gemäß Anspruch 4, wobei wenigstens ein Monomer f) ausgewählt ist aus C₁-C₆-(Meth)acrylaten.

6. Polymer gemäß einem der Ansprüche 1 bis 5, wobei wenigstens eine Verbindung d1) ausgewählt ist aus Polyether(meth)acrylaten, die mit C₈-C₂₂-Alkylgruppen terminiert sind.

7. Polymer gemäß einem der Ansprüche 1 bis 6, wobei das Molverhältnis der Verbindungen a) zu c) wenigstens 4:1 beträgt.

8. Polymer gemäß einem der Ansprüche 1 bis 7, umfassend als polymerisierte Einheiten
a) 65 bis 75 Gew.-% Acrylsäure,
b) 10 bis 25 Gew.-% N-Vinylpyrrolidon,
c) 0,5 bis 2 Gew.-% N-Vinylimidazol,
d) 2 bis 6 Gew.-% C₁₆-C₁₈-PEG-25-Methacrylat,
e) 0,5 bis 1,5 Gew.-% an wenigstens einem Vernetzungsmittel,
f) 2 bis 8 Gew.-% Methacrylsäure und 0,2 bis 1,0 Gew.-% Methylmethacrylat,
wobei sich die Gesamtmenge von a) bis f) auf 100 Gew.-% aufsummiert.

9. Kosmetische Zusammensetzung, umfassend ein Polymer gemäß einem der Ansprüche 1 bis 8.

10. Verfahren zum Modifizieren der Viskosität von wässrigen Zusammensetzungen, wobei das Verfahren das Zugeben eines Polymers gemäß einem der Ansprüche 1 bis 8 zu den wässrigen Zusammensetzungen umfasst.

## Revendications

1. Polymère comprenant, au titre de motifs polymérisés
a) entre 25 et 85 % en masse d'acide acrylique,
b) entre 10 et 60 % en masse de N-vinylpyrrolidone,
c) entre 0,5 et 10 % en masse d'au moins un monomère cationique,
d) entre 0,5 et 10 % en masse d'au moins l'un des composés d1) ou d2) 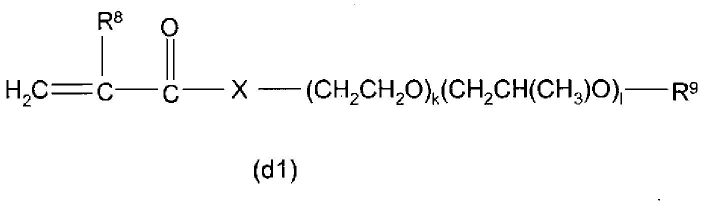
H₂C=CH-CH₂-O-(CH₂CH₂O)ₖ(CH₂CH(CH₃)O)ₗ-R⁹ (d2)
où
l'ordre des motifs oxyde d'alkylène est arbitraire, chacun des nombres k et l représente indépendamment de l'autre un entier compris entre 0 et 1000,
la somme de k et de l étant au moins égale à 5,
R⁸ représente un atome d'hydrogène ou un groupement alkyle en C₁-C₄,
R⁹ représente un groupement alkyle en C₈-C₃₀, alcényle en C₈-C₃₀ ou (alkyle en C₈-C₃₀)-aryle, et
X représente O ou un groupement de formule NR¹⁰, où R¹⁰ représente H ou un groupement alkyle, alcényle, cycloalkyle, hétérocycloalkyle, aryle ou hétéroaryle,
e) entre 0,01 et 2 % en masse d'au moins un agent de réticulation,
f) entre 0 et 30 % en masse de monomères supplémentaires différents de a) à e), le total des proportions de a) à f) atteignant 100 % en masse.

2. Polymère conforme à la revendication 1, où au moins un monomère cationique c) est choisi parmi les vinylimidazoles de formule générale (II) 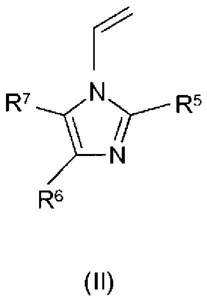 où chacun des radicaux R⁵ à R⁷ représente indépendamment des autres un atome d'hydrogène ou un groupement alkyle en C₁-C₄ ou phényle.

3. Polymère conforme à la revendication 2, où au moins l'un des monomères cationiques c) est le N-vinylimidazole.

4. Polymère conforme à l'une des revendications 1 à 3, où au moins l'un des monomères f) est choisi parmi l'acide méthacrylique et les esters d'acides mono et dicarboxyliques portant des insaturations α,β-éthyléniques avec des alcanols en C₁-C₈.

5. Polymère conforme à la revendication 4, où au moins l'un des monomères f) est choisi parmi les acrylates et les méthacrylates en C₁-C₆.

6. Polymère conforme à l'une des revendications 1 à 5, où au moins l'un des composés d1) est choisi parmi les acrylates et les méthacrylates de polyéther terminés par des groupements alkyle en C₈-C₂₂.

7. Polymère conforme avec des revendications 1 à 6, où le rapport molaire des composés a) à c) est au moins égal à 4:1.

8. Polymère conforme à l'une des revendications 1 à 7, comprenant au titre de motifs polymérisés
a) entre 65 et 75 % en masse d'acide acrylique,
b) entre 10 et 25 % en masse de N-vinylpyrrolidone,
c) entre 0,5 et 2 % en masse de N-vinylimidazole,
d) entre 2 et 6 % en masse de méthacrylate de PEG-25 en C₁₆-C₁₈,
e) entre 0,5 et 1,5 % en masse d'au moins un agent de réticulation,
f) entre 2 et 8 % en masse d'acide méthacrylique et entre 0,2 et 1,0 % en masse de méthacrylate de méthyle, le total des proportions de a) à f) atteignant 100 % en masse.

9. Composition cosmétique comprenant un polymère conforme à l'une des revendications 1 à 8.

10. Procédé de modification de la viscosité de compositions aqueuses, où ledit procédé comprend l'ajout d'un polymère conforme à l'une des revendications 1 à 8 auxdites compositions aqueuses.
